# EUROPEAN PATENT APPLICATION

(11) **EP 3 146 947 A1**
(43) Date of publication of application: **29.03.2017**
(21) Application number: 15186970.8
(22) Date of filing: 25.09.2015
(51) Int. Cl.: A61F 9/02

(54) **THREE-DIMENSIONAL STRUCTURE LENS GROUP FOR SKI AND SNOWBOARDING MASKS AND MASK PROVIDED WITH SAID GROUP**

(71) Applicant: WMT Asia Limited, Cheung Sha Wan, Kowloon, Hong Kong (HK)
(72) Inventor: DEPALO, Pasquale, Kowloon, Hong Kong (HK)
(74) Representative: Branca, Emanuela

(57) **Abstract**

Ski and snowboarding mask (10) comprising a frame (11) on one side (12) configured to couple with the user's face and on the other side configured to support a lens group (14) provided with at least one lens (15, 16), in which said lens group comprises a framework (17) configured to support said at least one lens (15, 16) and couple at the rear with said frame (11), said framework (17) and said at least one lens (15, 16) being made of the same material, so as to be able to undergo the same treatments also in assembled configuration.

## Description

The present invention refers to a lens group for ski and snowboarding masks.

In particular, the present invention refers to a lens group configured so that a single three-dimensional structure element is in view, i.e. a structure that is curved without solution of continuity.

In the field of the sports activity of skiing and snowboarding the use of masks with protective lenses is essential.

Usually snowboarding or ski masks comprise a frame with which two spaced lenses are coupled, one inner and one outer.

The frame is usually made from a flexible material so as to ensure that it adheres to the face, that it is capable of absorbing bangs and of maintaining the same characteristics at any temperature.

Indeed, the frame, subjected to very low temperatures during the aforementioned sports activities, must not become too rigid and thus more subject to breaking.

As stated earlier, the frame is coupled with a pair of lenses that are juxtaposed and spaced apart so as to ensure greater resistance to bangs and prevent the condensation effect caused by the thermal excursion between the heat of the face and the external temperature.

In order to best deal with any climatic situation, the lenses undergo anti-reflective treatments, known as "mirroring of the lens".

Currently, lenses for ski and snowboarding masks, usually made from acetate or polycarbonate, are produced in two ways.

The first way consists of starting from a sheet to then take care of the shaped shearing thereof.

The other technology consists of obtaining the lens from a so-called "injected block" that allows various radii of curvature to be made, both spherical and cylindrical. Finally, the lens thus obtained is then milled.

The assembly system of the lenses to the frame can take place in various ways.

One of the most conventional ones is the insertion of the lenses in a prearranged channel that extends along the entire profile of the frame ensuring the seal.

The new stylistic and market trends involve the possibility of having the lenses with view cut-off profile over the entire perimeter or at least partially.

This is made possible also by the progressive use of the injected lenses that, due to their rigidity and structure, allow insertion in the frame only in some points.

From such a prior art, the purpose of the present invention is to make a lens group for ski and snowboarding masks that is an alternative to those that are known.

In particular, the purpose of the present invention is to make a lens group to be coupled with the frame that has a structure that is not two-dimensional, typical of sheets and of glass sheets, but rather a three-dimensional structure as if the lens group were a single volume enveloping the frame.

This would indeed allow a mask to be obtained without view cut-off profiles or sharp effects with, contrarily, a perception of a soft and elusive object. In general, these purposes are accomplished thanks to a lens group for ski and snowboarding masks comprising a pair of shaped lenses assembled to a framework moulded with the same material as the lenses.

Such a group, to then be coupled with the frame to make the mask, thus acquires an appearance as if it was a single and three-dimensional element enveloping the frame.

This effect is further accentuated thanks to the fact that framework and lenses, being of the same material, can undergo the same mirroring or chromium plating processes with the elements already assembled.

This would make it possible to have the same chromatic effect between lenses and framework without solution of continuity emphasising the uniqueness and the three-dimensional nature of the group to a greater extent. Further characteristics of the invention are highlighted by the attached claims.

The characteristics and advantages of a lens group for ski and snowboarding masks according to the present invention will be clearer from the following description, given as an example and not for limiting purposes, referring to the attached schematic drawings, in which:
- figure 1 shows a perspective view of a ski and snowboarding mask provided with the lens group according to the present invention;

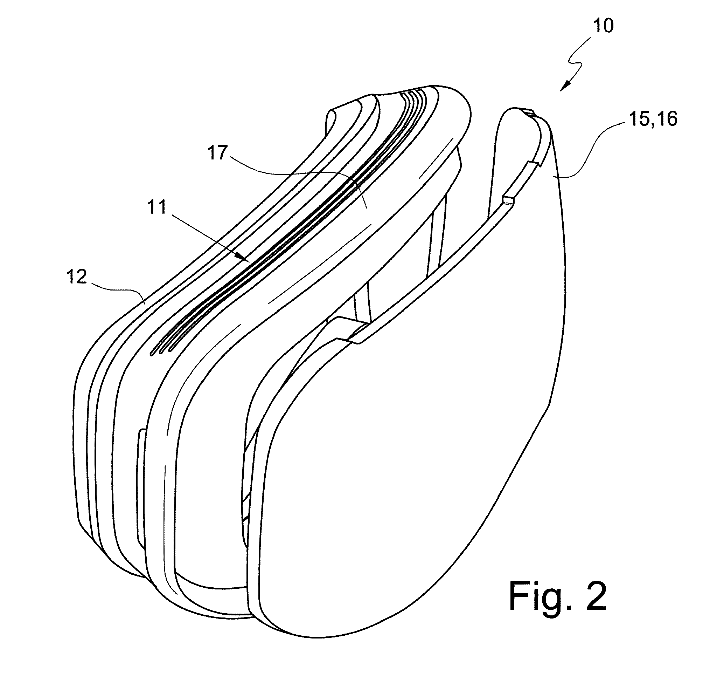

- figure 2 shows an exploded view of the mask of figure 1;

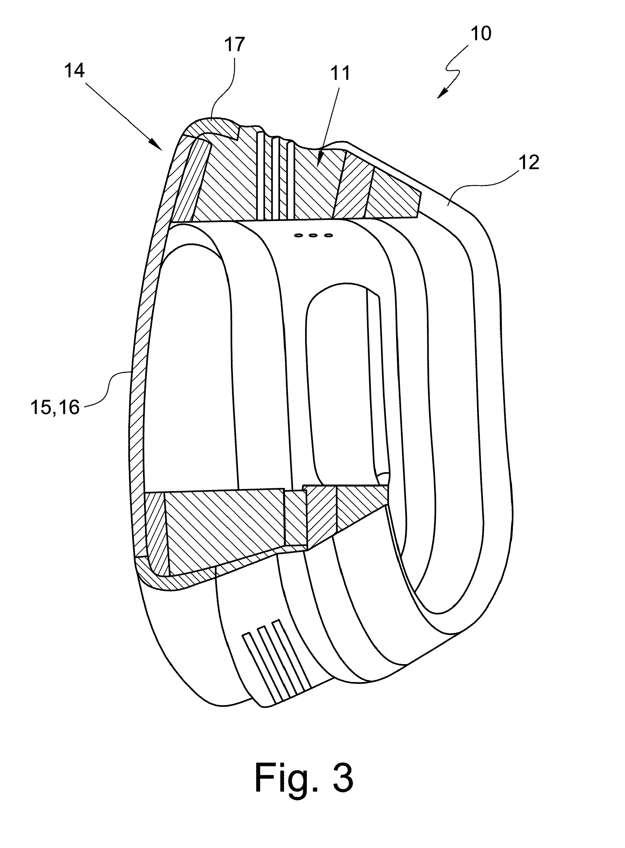

- figure 3 shows a section of the mask of figure 1;

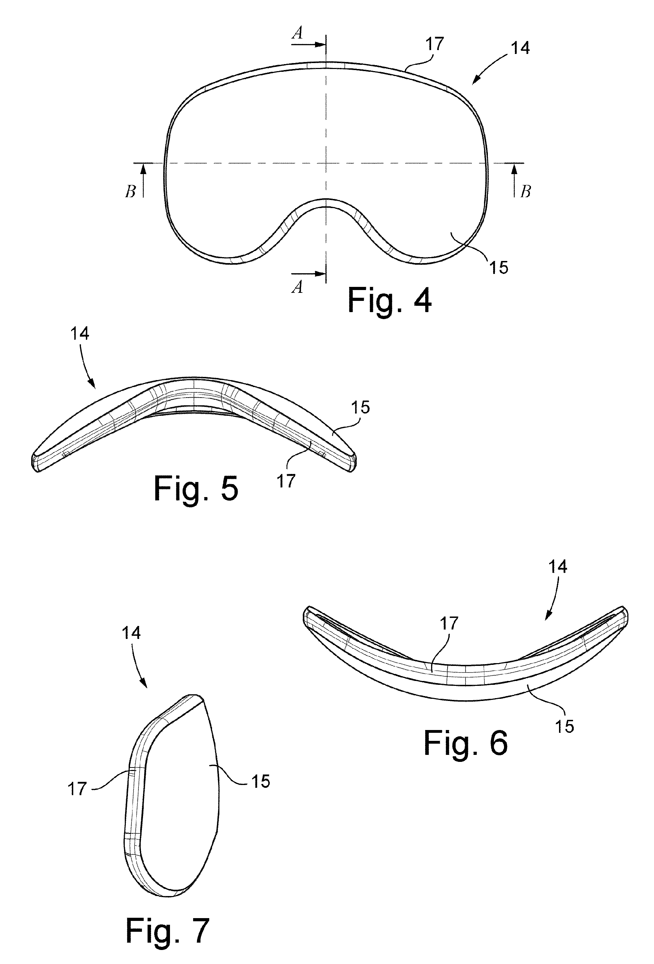

- figures 4-7 show views of an example of a lens group according to the present invention;
- figures 8 and 9 show sections of figure 4 respectively along the section lines B-B and A-A.

With reference to the figures, an embodiment of a lens group for ski and snowboarding masks according to the present invention is shown with 14.

Reference numeral 10 indicates the mask as a whole, i.e. comprising a frame 11 configured on one side 12 to couple with the face of the user and on the other side to support a lens group 14 of the present invention. Such a lens group 14 comprises at least one lens 15, 16, preferably an outer lens 15 and an inner lens 16, and a perimeter outer framework 17 configured to stably support such at least one lens 15, 16.

The framework is also configured to couple at the rear, for example along a rear extension 20, with the frame 11.

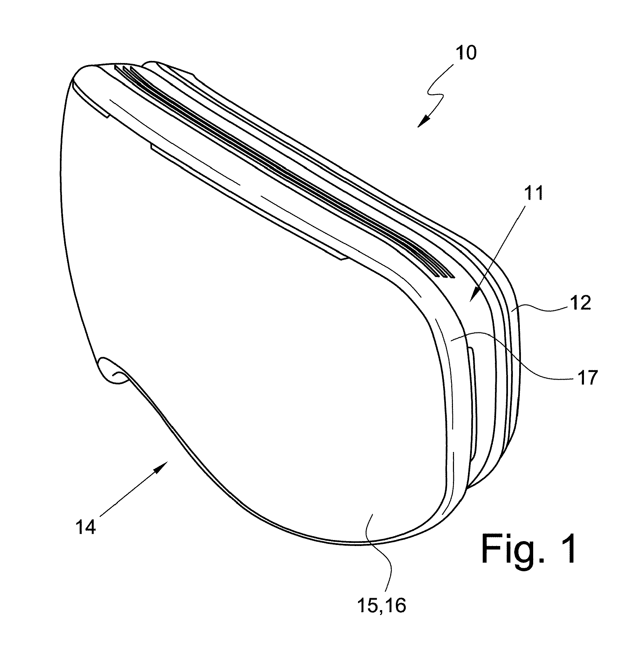

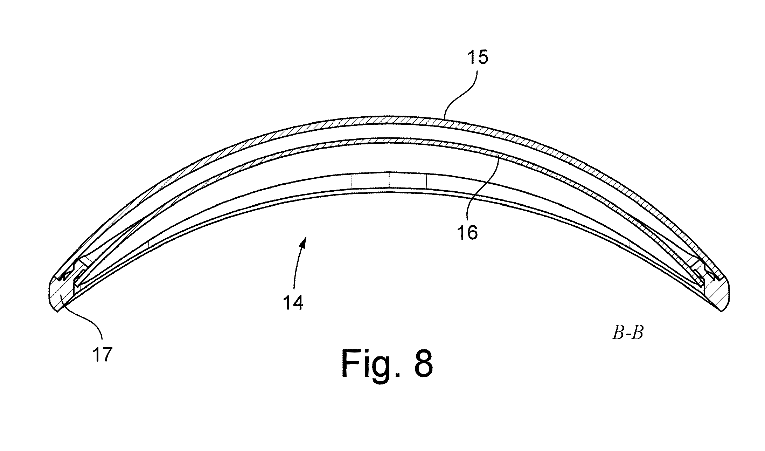

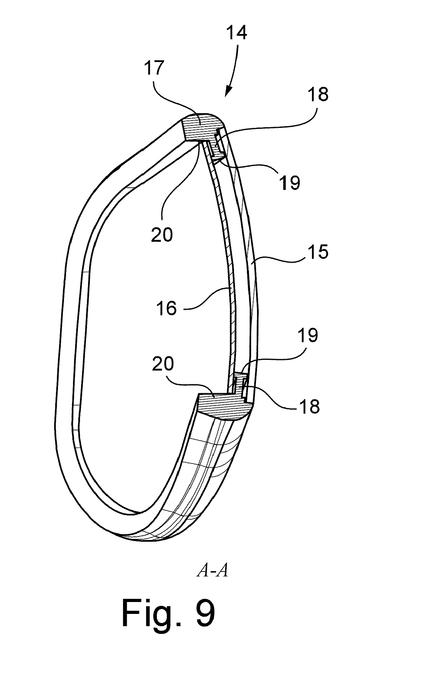

According to the invention, both the framework 17, and the at least one lens 15, 16 are made of the same material so as to be able to undergo the same treatments also in the already-assembled configuration. As can be seen in the sections of figures 8 and 9, and as indicated earlier, the lens group 14 preferably comprises a pair of lenses 15, 16 coupled in the framework 17 in mutually facing and separated position. As can be seen in figure 1, the outer lens 15 is coupled with the framework 17 so as to make a continuous curved outer profile for partial three-dimensional envelopment of the frame 11.

In order to keep the lenses 15, 16 stably in position, the framework 17 can comprise a rod element 18 that partially extends in the gap defined by the lenses 15, 16.

In this case the rod element 18 can comprise an enlarged head 19 for contact with the lenses 15, 16. Preferably, both the framework 17, and the outer lens 15, are made by injection. Preferably, the outer lens 15 is also spherical or toric in shape.

The inner lens 16, having the same shape as the outer one 15, is made from cellulose acetate or another material with anti-misting treatment.

The framework 17 and the pair of lenses 15, 16 are coupled together through a bi-adhesive gasket, viscous sealant or by ultrasound welding.

It has thus been seen that a lens group for ski and snowboarding masks according to the present invention achieves the purposes outlined earlier, offering to the market a valid alternative to known lens groups. Indeed, the lens group of the present invention when coupled with the relative frame makes the mask provided with a particular appearance as if it was a single three-dimensional element enveloping the frame itself. This effect is further accentuated thanks to the fact that framework and lenses, being made from the same material, can undergo the same mirroring or chromium plating processes with elements that are even already assembled emphasising the uniqueness and the three-dimensional nature of the group to a greater extent. The lens group for ski and snowboarding masks of the present invention thus conceived can undergo numerous modifications and variants, all of which are covered by the same inventive concept; moreover, all of the details can be replaced by technically equivalent elements. In practice, the materials used, as well as their sizes, can be of any type depending on the technical requirements.

In the detail of the invention the shape of the framework, combined with the lens, can be unconstrained by the shape that the frame will have, making infinite solutions possible both in terms of shape and of volumes for the ski and snowboarding masks as a whole.

## Claims

1. Ski and snowboard mask (10) comprising a frame (11) on one side (12) configured to couple with the user's face and on the other side configured to support a lens group (14) equipped with at least one lens (15, 16), **characterised in that** said lens group comprises a framework (17) configured to perimetrically support said at least one lens (15, 16) and to couple at the back with said frame (11), said framework (17) and said at least one lens (15, 16) being made of the same material, so as to be able to undergo the same treatments even in assembled configuration.

2. Mask (10) according to claim 1 **characterised in that** it comprises a pair of lenses (15, 16), outer (15) and inner (16) respectively, coupled with said framework (17) in a position facing one another and separated.

3. Mask (10) according to claim 2 **characterised in that** the outer lens (15) of said pair of lenses (15, 16) is coupled with said framework (17) so that said lens group (14) makes a continuous curved outer profile of partial three-dimensional envelopment of said frame (11).

4. Mask (10) according to any one of the previous claims **characterised in that** said framework (17) externally envelops the entire outer profile of said lenses (15, 16) and comprises a rod element (18) for keeping said lenses (15, 16) in separated position that partially extends in the gap defined by said lenses (15, 16).

5. Mask (10) according to claim 4, **characterised in that** said rod element (18) comprises an enlarged head (19) for contact with said lenses (15, 16).

6. Mask (10) according to any one of the previous claims **characterised in that** said framework (17) and said outer lens (15) are made by injection, said inner lens (16) being treated to prevent misting.

7. Mask (10) according to any one of the previous claims **characterised in that** said framework (17) and said pair of lenses (15, 16) are coupled together through a bi-adhesive gasket, viscous sealant or by ultrasound welding.

8. Lens group (14) for a ski and snowboarding mask (10) of the type able to be coupled by a frame (11) and comprising at least one lens (15, 16) and a framework (17) configured to support said at least one lens (15, 16) and couple at the rear with said frame (11), said framework (17) and said at least one lens (15, 16) being made of the same material, so as to be able to undergo the same treatments in assembled configuration.

9. Lens group (14) according to claim 8 **characterised in that** it comprises a pair of lenses (15, 16) coupled with said framework (17) in mutually facing and separated position.

10. Lens group (14) according to claim 9 **characterised in that** the outer lens (15) of said pair of lenses (15, 16) is coupled with said framework (17) so that said lens group (14) makes a continuous curved outer profile for partial three-dimensional envelopment of said frame (11).

11. Lens group (14) according to any one of the previous claims 8 to 10 **characterised in that** said framework (17) externally envelops the entire outer profile of said lenses (15, 16) and comprises a rod element (18) for keeping said lenses (15, 16) in separated position that partially extends in the gap defined by said lenses (15, 16).

12. Lens group (14) according to claim 11 **characterised in that** said rod element (18) comprises an enlarged head (19) for contact with said lenses (15, 16).

13. Lens group (14) according to any one of the previous claims 8 to 12 **characterised in that** said framework (17) and said outer lens (15) are made by injection, said inner lens (16) being treated to prevent misting.

14. Lens group (14) according to any one of the previous claims 8 to 13 **characterised in that** said framework (17) and said pair of lenses (15, 16) are coupled together through a bi-adhesive gasket, viscous sealant or by ultrasound welding.
